(19) Europäisches Patentamt
European Patent Office
Office européen des brevets

(11) Publication number: **0 136 831 B1**

# (12) EUROPEAN PATENT SPECIFICATION

(45) Date of publication of patent specification: **09.11.88**

(51) Int. Cl.⁴: **C 07 H 17/00,** A 61 K 31/70

(21) Application number: **84306009.6**

(22) Date of filing: **03.09.84**

## (54) Azahomoerythromycin B derivatives and intermediates thereof.

(30) Priority: **06.09.83 US 529827**

(43) Date of publication of application:
**10.04.85 Bulletin 85/15**

(45) Publication of the grant of the patent:
**09.11.88 Bulletin 88/45**

(84) Designated Contracting States:
**AT BE CH DE FR GB IT LI LU NL SE**

(56) References cited:
**EP-A-0 092 348**
**EP-A-0 101 186**
**EP-A-0 109 253**
**GB-A-2 047 247**
**GB-A-2 094 293**
**US-A-4 382 085**

(73) Proprietor: **PFIZER INC.**
**235 East 42nd Street**
**New York, N.Y. 10017 (US)**

(72) Inventor: **Bright, Gene Michael**
**329 Tyler Avenue**
**Groton Connecticut (US)**
Inventor: **Hauske, James Robert**
**15 Westchester Road**
**East Lyme Connecticut (US)**

(74) Representative: **Wood, David John et al**
**Pfizer Limited Ramsgate Road**
**Sandwich Kent CT13 9NJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention is concerned with anti-bacterial 9-deoxo-9a-methyl-9a-aza-9a-homoerythromycin B; pharmaceutically-acceptable salts thereof, and an intermediate useful in the preparation thereof from erythromycin B.

Erythromycin B is a well-known macrolide antibiotic, having the formula (I).

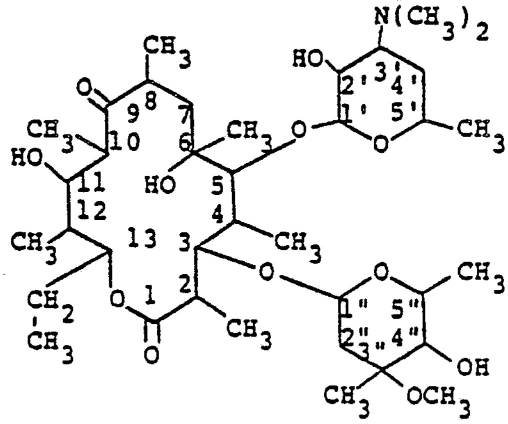

(I)

The present therapeutic compound of normal 4''-configuration of the formula (II) below is structurally related to the previously reported erythromycin A derivative of the formula (III), the subject of

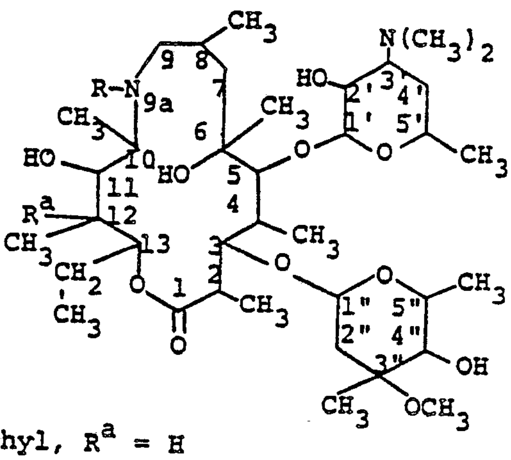

(II) R = methyl, $R^a$ = H

(III) R = methyl, $R^a$ = OH

(IV) R = $R^a$ = H

(V) R = H, $R^a$ = OH

British Patent Application No. 2,094,293, as well as European Patent Application publication No. 101,186. In those applications, the compound of the formula (III) is named as the N-methyl derivative of "11-aza-10-deoxo-10-dihydroerythromycin A", a name coined earlier by Kobrehel *et al.,* U.S. Patent 4,328,334 for the precursor compound of the formula (V). For the letter ring expanded (homo), aza (nitrogen substituted for carbon) erythromycin A derivative, we prefer the name 9-deoxo-9a-aza-9a-homoerythromycin A. That compound could also be named as a 10-aza-14-hexadecanolide derivative.

The present invention encompasses the anti-bacterial compound 9-deoxo-9a-methyl-9a-aza-9a-homoerythromycin N having the formula (II); pharmaceutically-acceptable salts thereof and pharmaceutical compositions thereof.

The present therapeutic compound shows a relatively broad spectrum of antibacterial activity which includes erythromycin A susceptible organisms and, in addition, many Gram negative microorganisms resistant to erythromycin A. This compound is of especial value in the oral treatment of susceptible bacterial infections in mammals.

The present invention also encompasses an intermediate useful in the synthesis of 9-deoxo-9a-methyl-9a-aza-9a-homoerythromycin B (II), namely 9a-aza-9a-homoerythromycin B.

The antibacterial compound of the present invention, 9-deoxo-9a-methyl-9a-aza-9a-homoerythromycin B (II), is readily prepared from erythromycin B by chemical process steps as follows:

(A) oxime formation

(B) ring expansion, with introduction of 9a-nitrogen;

(C) removal of the 9-oxo group; and

(D) 9a-N-methylation.

The antibacterial compound is prepared directly by the sequence (A) (B) (C) (D). The various intermediates and final product are isolated by standard manipulative methods (e.g., extraction, precipitation, evaporation, chromatography, crystallization).

Oxime formation is accomplished by reacting erythromycin B with hydroxylamine or preferably, a hydroxylamine salt such as the hydrochloride. Under presently preferred conditions, at least one molar equivalent, usually an excess, e.g., 10—30 equivalents, of the hydroxylamine is employed; in an excess of a weakly basic, tertiary amine (preferably pyridine) as solvent; at a temperature in the range 20—80°C, preferably in the range of 30—70°C.

The resulting erythromycin B oxime is rearranged to 9a-aza-9a-homoerythromycin B [having the above formula (IV), but with a keto group at the 9-position] *via* a Beckman rearrangement. The preferred conditions employ an excess (e.g., 2—4 molar equivalents) of an organic sulfonyl chloride, preferably p-toluenesulfonyl chloride, which is reacted with the oxime (as free base or as an acid salt) in a mixture of a lower ketone (e.g., methyl ethyl ketone, acetone) and water containing a large molar excess of sodium bicarbonate, at a temperature of 0—50°C, preferably at 0—30°C.

The C—9 amide carbonyl is then conveniently reduced to the corresponding dihydro derivative, i.e., 9-deoxo-9a-aza-9a-homoerythromycin B (IV) by reduction with sodium-borohydride (preferably in excess to force the reaction to completion in a reasonable time period, but with at least two equivalents). The reduction is carried out in a suitable protic solvent, such as a lower alkanol (preferably methanol) at 0—50°C.

Final methylation to yield the compound (IV) is acccomplished by reductive methylation, using formaldehyde in the presence of a reducing agent, such as hydrogen and a noble metal catalyst, sodium cyanoborhydride, or, preferably, formic acid. The reaction is preferably carried out with at least one equivalent each of formaldehyde and formic acid in a reaction-inert solvent at 20—100°C, preferably 30—60°C. The preferred solvent is chloroform. As used herein, the expression "reaction-inert solvent" refers to any solvent which does not interact with reagents, intermediates or product in a manner which adversely affects the yield of the desired product.

Since the compound (II) of the present invention contains two basic nitrogen atoms, pharmaceutically acceptable mono and di acid addition salts are formed by contacting the corresponding free base of (II) with substantially one equivalent of the acid or with at least two equivalents of the acid, as appropriate. Salts are generally formed by combining the reagents in a reaction-inert solvent; if the salt does not precipitate directly, it is isolated by concentration and/or addition of a non-solvent. Suitable pharmaceutically acceptable acid addition salts include, but are not restricted to those with HCl, HBr, $HNO_3$, $H_2SO_4$, $HO_2CCH_2CH_2CO_3H$, *cis*- and *trans*-$HO_2CCHCHCO_2H$, $CH_3SO_3H$ and *p*-$CH_3C_6H_4SO_3H$.

The antibacterial activity of the compound of the formula (II) is demonstrated by measuring minimum inhibitory concentrations (MIC's) in µg/ml against a variety of microorganisms in brain heart infusion (BHI) broth. Generally twelve 2 fold dilutions of the test compound are employed, with initial concentration of the test drug being in the range of 50 to 200 µg/ml. The susceptibility (MIC) of the test organism is accepted as the lowest concentration of compound capable of producing complete inhibition of growth as judged by the naked eye. A same-day comparison of the activity of 9-deoxo-9a-methyl-9a-aza-9a-homomerythromycin B (II) with that of erythromycin A and 9-deoxo-9a-methyl-9a-aza-9a-homoerythromycin A controls is shown in Table I.

TABLE I

*In vitro* Activity of Compound (II) and Related Compounds

| Microorganism | | MIC Values (µg/ml) (1) | (2) | (3) |
|---|---|---|---|---|
| *Staph. aur.* | 005 | 0.05 | 0.20 | 0.39 |
| | 052 | 0.10 | 0.39 | 0.39 |
| | 400 | 6.25 | 25 | (a) |
| *Staph. epi.* | 111 | 0.05 | 0.10 | 0.20 |
| *Strep. faec.* | 006 | 0.39 | 1.56 | 1.56 |
| *Strep. pyog.* | 203 | 0.025 | 0.025 | 0.025 |
| *E. coli* | 125 | (a) | 12.5 | 50 |
| | 129 | (a) | 6.25 | 25 |
| | 266 | (a) | 6.25 | 25 |
| | 470 | 1.56 | 0.78 | 0.39 |
| *Kleb. pn.* | 009 | (a) | 12.5 | 50 |
| | 031 | (a) | 12.5 | 50 |
| *Kleb. oxy.* | 024 | (a) | 25 | 50 |
| *Past. mult.* | 001 | 0.78 | 0.10 | 0.10 |
| *Serr. mar.* | 017 | (a) | 50 | (a) |
| *Neiss. sic.* | 000 | 3.12 | 0.39 | 0.39 |
| *Ent. aerog.* | 040 | (a) | 12.5 | (a) |
| *Ent. cloac.* | 009 | (a) | 25 | (a) |
| *Prov. strua.* | 013 | (a) | 50 | (a) |
| *H. influ.* | 012 | 3.12 | - | 1.56 |
| | 036 | 3.12 | 0.78 | 3.12 |
| | 038 | 1.56 | 0.78 | 3.12 |
| | 042 | 3.12 | 0.78 | 1.56 |
| | 051 | 3.12 | 1.56 | 3.12 |
| | 073 | 3.12 | 0.78 | 0.20 |
| | 078 | 1.56 | 0.39 | 0.78 |
| | 081 | 3.12 | 0.78 | 1.56 |

(a) greater than 50

(1) Erythromycin A

(2) 9-Deoxo-9a-methyl-9a-aza-9a-homoerythromycin A

(3) 9-Deoxo-9a-methyl-9a-aza-9a-homoerythromycin B

Additionally, the compound (II) is tested *in vivo* by the well-known mouse protection test, or by a microbiological (bioassay) determination of serum levels in a variety of mammals (e.g., mouse, rat, dog). Using mice as the test species, compound (II) has been shown to be exceptionally well absorbed after oral dosage, providing exceptionally high and long lasting serum levels.

For the treatment of systemic infections in animals, including man, caused by susceptible microorganisms, the compound (II) is dosed at a level of 2.5—100 mg/kg per day, preferably 5—50 mg/kg/day, in divided doses, or preferably by a single daily dose. Variation in dosage will be made depending upon the individual and upon the susceptibility of the microorganism. These compounds are dosed orally or parenterally, the preferred route being oral. The susceptibility of microorganisms isolated in the clinics is routinely tested in clinical laboratories by the well-known disc-plate method. The compound (II) is generally the compound of choice when it shows a relatively large zone of inhibition against the bacteria causing the infection to be treated.

Preparation of optional dosage forms will be by methods well known in the pharmaceutical art. For oral administration, the compounds are formulated alone or in combination with pharmaceutical carriers such as inert solid diluents, aqueous solutions or various non-toxic organic solvents in such dosage forms as gelatin capsules, tablets, powders, lozenges, syrups and the like. Such carriers include water, ethanol, benzyl alcohol; glycerin, propylene glycol, vegetable oils, lactose, starches, talc, gelatins, gums and other well known carriers. The parenteral dosage forms required for the above systemic use are dissolved or suspended in a pharmaceutically-acceptable carrier such as water, saline, sesame oil and the like. Agents which improve the suspendability and dispersion qualities can also be added.

For the topical treatment of superficial infections in animals, including man, caused by susceptible microorganisms, the compound (II) is formulated by methods well known in the pharmacist's art into lotions, ointments, creams, salves, gels, or the like at concentrations in the range 5—200 mg/cc of the dosage form, preferably in the range 10—100 mg/cc. The dosage form is applied at the site of infection *ad libitum,* generally at least once a day.

The present invention is illustrated by the following examples. However, it should be understood that the invention is not limited to the specific details of these examples.

### Example 1

Erythromycin B Oxime

Erythromycin B (2.0 g, 2.78 mmoles) and hydroxylamine hydrochloride (1.45 g, 20.9 mmoles) were combined in 40 ml pyridine and stirred at 60° for 6 hours. Additional hydroxylamine hydrochloride (0.725 g, 10.4 mmoles) was added and stirring at 60° continued for 16 hours. The reaction was poured into 1:1 $H_2O$:$CH_2Cl_2$ and the pH adjusted to 10 with dilute NaOH. The aqueous layer was separated and washed with fresh $CH_2Cl_2$. The organic layers were combined, washed with $H_2O$ and then saturated NaCl, dried and stripped with toluene chase. The resulting foam (2.13 g) was dissolved in 20 ml hot 3:1 hexane:$CHCl_3$ and partially boiled down until crystallization began. After stirring at room temperature, crystalline title product was recovered by filtration, 1.9 g, identical with the known $CHCl_3$ solvate of erythromycin B oxime [J. Org. Chem., Vol. 39, pp. 2492—2494 (1974)].

### Example 2

9a-Aza-9a-homoerythromycin B

Title product of the preceding Example (1.65 g, 1.93 mmoles) was dissolved in 15 ml acetone. With stirring, $NaHCO_3$ (894 mg, 10.6 mmoles) and then 6.5 ml $H_2O$ were added, the mixture was cooled to 5°, *p*-toluenesulfonyl chloride (738 mg, 3.87 mmoles) was added, and the mixture stirred 1.5 hours at that temperature. The mixture was poured into 1:1 $H_2O$:$CH_2Cl_2$ and the pH adjusted to 5.3 with dilute HCl. The aqueous layer was separated, extracted with fresh $CH_2Cl_2$ and adjusted to 9.7 with dilute NaOH, and twice more extracted with fresh portions of $CH_2Cl_2$. The basic extracts were combined, dried and stripped to yield title product as a white foam, 1.27 g.

$C^{13}$nmr ($CDCl_3$) 177.3, 163.4, 102.8, 95.3, 87.1, 78.8, 77.7, 77.5, 76.1, 72.6, 71.9, 70.4, 68.6, 65.3, 55.9, 49.2, 44.7, 42.6, 41.7, 40.1, 35.2, 34.6, 25.3, 24.8, 21.4, 21.1, 18.0, 17.9, 15.4, 13.7, 10.1, 9.7, 9.4.

### Example 3

9-Deoxo-9a-aza-9a-homoerythromycin B

Title product of the preceding Example (1.16 g, 1.59 mmoles) in 15 ml methanol was cooled to 5°. $NaBH_4$ (604 mg, 15.9 mmoles) was added over 2 minutes and the mixture stirred 1 hour at 5°. The reaction mixture was poured into 1:1 $H_2O$:$CH_2Cl_2$ and the aqueous layer separated and extracted with fresh $CH_2Cl_2$. The organic layers were combined, dried and stripped to yield title product as a white foam, 1.08 g, used directly in the next step.

### Example 4

9-Deoxo-9a-methyl-9a-aza-9a-homoerythromycin B

Title product of the preceding Example (1.0 g, 1.39 mmoles), 37% HCHO (0.106 ml, 1.42 mmoles) and $HCO_2H$ (0.051 ml, 1.40 mmoles) were combined in 15 ml $CHCl_3$ and stirred at 45—50° for 68 hours. The reaction mixture was poured into 1:1 $H_2O$:$CHCl_3$, and after adjusting the pH to 9.7, title product further isolated as a white foam according to the preceding Example, 884 mg.

$C^{13}$nmr ($CDCl_3$) 179.0, 103.7, 95.9, 84.4, 79.4, 79.2, 76.4, 74.8, 74.0, 72.0, 69.6, 66.7, 63.3, 50.4, 45.8, 42.9, 41.4, 39.4, 38.2, 30.0, 28.5, 28.2, 25.8, 23.1, 22.7, 22.5, 19.3, 15.5, 11.3, 10.6, 10.5, 8.7.

## Claims for the Contracting States: BE CH DE FR GB IT LI LU NL SE

1. 9-Deoxo-9a-methyl-9a-aza-9a-homoerythromycin B or a pharmaceutically-acceptable salt thereof.

2. A pharmaceutical composition which comprises an antibacterial amount of the compound of claim 1 or a pharmaceutically-acceptable salt thereof, and a pharmaceutically-acceptable carrier.

3. 9a-Aza-9a-homoerythromycin B.

**Claims for the Contracting State: AT**

1. A process for the preparation of 9-deoxo-9a-methyl-9a-aza-9a-homoerythromycin B or a pharmaceutically-acceptable salt thereof which is characterised by the reaction of 9-deoxo-9a-aza-9a-homoerythromycin B with formaldehyde in the presence of a reducing agent and, if desired, conversion of the product to a pharmaceutically-acceptable salt.

2. The process of claim 1 wherein the reducing agent is formic acid.

3. A process for the preparation of 9a-aza-9a-homoerythromycin B, which is characterised by the rearrangement of erythromycin B oxime in the presence of an organic sulfonyl chloride and sodium bicarbonate.

4. The process of claim 3 wherein the organic sulfonyl chloride is *p*-toluenesulfonyl chloride.

5. 9-Deoxo-9a-methyl-9a-aza-9a-homoerythromycin B or 9a-aza-9a-homoerythromycin B.

**Patentansprüche für die Vertragsstaaten: BE CH DE FR GB IT LI LU NL SE**

1. 9-deoxo-9a-methyl-9a-aza-9a-homoerythromycin B oder ein pharmazeutisch annehmbares Salz hievon.

2. Pharmazeutisch Zusammensetzung, die einen antibakteriellen Anteil der Verbindung nach Anspruch 1 oder eines pharmazeutisch annehmbaren Salzes hievon und einen pharmazeutisch annehmbaren Träger umfaßt.

3. 9a-Aza-9a-homoerythromycin B.

**Patentansprüche für den Vertragsstaat: AT**

1. Verfahren zur Herstellung von 9-Deoxo-9a-methyl-9a-aza-9a-homoerythromycin B oder eines pharmazeutisch annehmbaren Salzes hievon, gekennzeichnet durch die Umsetzung von 9-Deoxo-9a-aza-9a-homoerythromycin B mit Formaldehyd in Anwesenheit eines Reduktionsmittels und, wenn gewünscht, überführen des Produktes in ein pharmazeutisch annehmbares Salz.

2. Verfahren nach Anspruch 1, worin das Reduktionsmittel Ameisensäure ist.

3. Verfahren zur Herstellung von 9a-Aza-9a-homoerythromycin B, gekennzeichnet durch die Umlagerung von Erythromycin B-Oxim in Anwesenheit eines organischen Sulfonylchlorids und von Natriumbicarbonat.

4. Verfahren nach Anspruch 3, worin das organische Sulfonylchlorid p-Toluolsulfonylchlorid ist.

5. 9-Deoxo-9a-methyl-9a-aza-9a-homoerythromycin B oder 9-Aza-9a-homoerythromycin B.

**Revendications pour les Etats contractants: BE CH DE FR GB IT LI LU NL SE**

1. La 9-déoxo-9a-méthyl-9a-aza-9a-homoérythromycin B ou un sel pharmaceutiquement acceptable de ce composé.

2. Composition pharmaceutique, qui comprend une quantité antibactérienne du composé suivant la revendication 1, ou d'un sel pharmaceutiquement acceptable de ce composé, et un support acceptable du point de vue pharmaceutique.

3. La 9a-aza-9a-homoérythromycine B.

**Revendications pour l'Etat contractant: AT**

1. Procédé de préparation de la 9-déoxo-9a-méthyl-9a-aza-9a-homoérythromycine B ou d'un sel pharmaceutiquement acceptable de ce composé, qui est caractérisé par la réaction de la 9-déoxo-9a-aza-9a-homoérythromycine B avec le formaldéhyde en présence d'un agent réducteur et, le cas échéant, la transformation du produit en un sel pharmaceutiquement acceptable.

2. Procédé suivant la revendication 1, dans lequel l'agent réducteur est l'acide formique.

3. Procédé de préparation de la 9a-aza-9a-homoérythromycine B, qui est caractérisé par la transposition de l'oxime de l'érythromycine B en la présence d'un chlorure de sulfonyle organique et de bicarbonate de sodium.

4. Procédé suivant la revendication 3, dans lequel le chlorure de sulfonyle organique est le chlorure de p-toluènesulfonyle.

5. La 9-déoxo-9a-méthyl-9a-aza-9a-homoérythromycine B ou la 9a-aza-9a-homoérythromycine B.